# EUROPEAN PATENT SPECIFICATION

(11) **EP 0 639 962 B1**
(45) Date of publication and mention of the grant of the patent: **14.04.1999**
(21) Application number: 92911065.8
(22) Date of filing: 30.04.1992
(51) Int. Cl.: A61F 11/06, G10K 11/16, G10K 11/178

(54) **HANDS FREE NOISE CANCELING HEADSET**
GERÄUSCHUNTERDRÜCKUNGSSYSTEM
CASQUE MAINS LIBRES A SUPPRESSION DE BRUIT

(43) Date of publication of application: 01.03.1995
(73) Proprietor: NOISE CANCELLATION TECHNOLOGIES, INC., Linthicum, MD 21090 (US)
(72) Inventor: CLAYBAUGH, Dave, Linthicum, MD 21090 (US); DENENBERG, Jeffrey, Stamford, CT 06901 (US)
(74) Representative: Dauster, Hanjörg, Dipl.-Ing.
(86) International application number: US9203441
(87) International publication number: WO9321876

(56) References cited:
- US-A- 2 972 018
- US-A- 4 494 074
- US-A- 4 677 677
- US-A- 4 736 432
- US-A- 4 953 217
- US-A- 4 977 600
- US-A- 5 001 763
- US-A- 5 033 082
- US-A- 5 091 954

## Description

The invention relates to a hands-free active noise canceling headset comprising clamping means adapted to secure the headset to the user's head, said headset including a speaker means and a microphone means operatively connected to a controller means adapted to produce an anti-noise signal to said speaker means.

US-A-5,001,763 discloses an active noise cancellation headset provided with a boom microphone, two input transducers and two speakers asigned to the headsets. The boom microphone is to pick up the user's speech for communication producing a signal which represents the user's speech and the surrounding noise from the environment superimposed. The input transducers are part of an active noise cancellation mechanism in the headset. They are positioned opposite to the outlet of the speaker mechanism in the headset so that they are subjected only to sound originating from the environment, but not exposed to sound originating from the speakers. A control unit is provided for amplifying and, if desired, for inverting the signals of the input transducers so that the speakers in the headset can be provided with a signal which is either amplifying sound picked up by the input transducers at a gain beyond the minimum level heard by the ear or which is canceling noise of the surrounding environment.

From US-A-4,667,667 an active acoustic attenuation system provided for attenuating an undesirable output acoustic wave in a duct or plant is known. This is achieved by introducing a canceling acoustic wave from an omni-directional speaker at the output of the duct or plant. By an adapted filter means connected to an input microphone at the input of the duct or plant and to an error microphone at the output of the duct or plant, which may comprise one or several LMS (Least Mean Square) filters, the duct or plant is modelled with an adapted filter model. This adapted filter means produces a correction signal based on the input microphone signal and the error microphone signal and feeds it to the speaker to introduce canceling sound or acoustic waves such that the error signal of the error microphone approaches a given value such as zero.

In the area of active noise cancellation headsets, the noisy environment often encountered in the link with communication systems causes lack of intelligibility on the outward communications path. Consequently another in-wire cancellation channel to enhance the intelligibility is required. This additional processing channel adds to the cost of the headset. "In-wire" cancellation refers to any of the active control approaches that performs the cancellation of noise in a manner such as that described in U.S. Patent Application No. 07/421 759, which is hereby incorporated by reference therein.

The instant invention contemplates the use of the headset residual microphones to pick up the user's speech for communications. It utilizes two seperate noise canceling systems both having a residual microphone located at the ear to sense any noise remaining. They also use a controller which synthesizes the anti-noise signal and a headset driver to deliver the anti-noise signal to the ear vicinity. The algorithm used is the one described in US-Patent No. 5,105,377, which is a digital virtual earth algorithm which develops a reference signal by subtracting an equalized anti-noise signal of its own from the residual signal.

Accordingly an object of this invention is to provide a hands-free active noise canceling headset in which the residual microphone is used to pick-up the user's speech for communication. This object is achieved by a hands-free active noise canceling headset having the features of claim 1. In such a headset the outward communication path can be handled by microphones in the earpieces.

A further object of this invention is to provide an active noise cancellation headset with improved voice intelligibility on the outward communication path. This object is achieved by a headset having the features of claim 5.

These and other objects will become apparent when reference is had to the accompanying description and drawings in which:
Fig. 1 is diagrammatic view of the headset of this invention and the circuitry therefore.
Fig. 2 shows a circuit for using residual microphones for communications

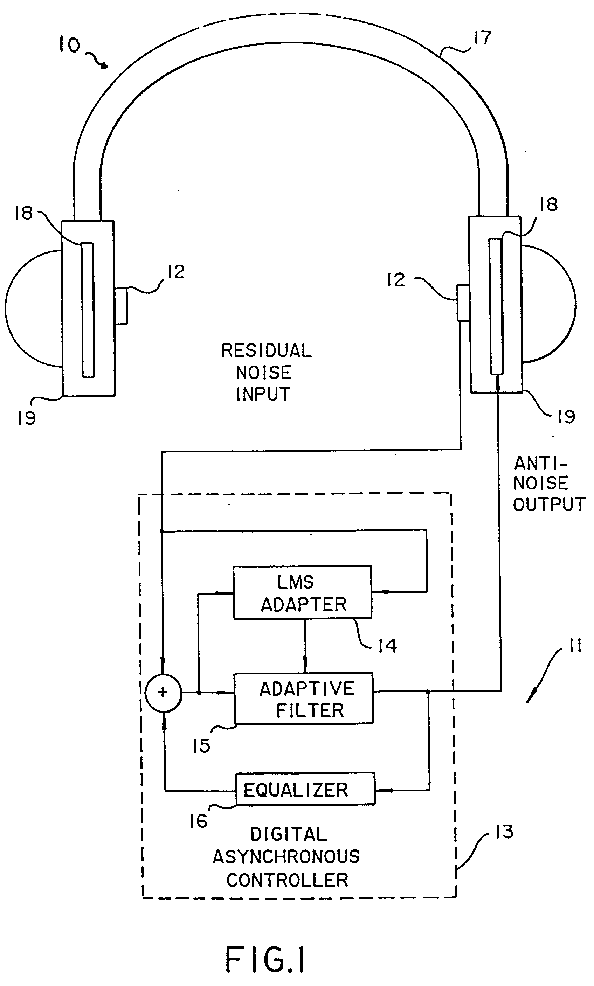

An active noise canceling headset system is shown in Fig. 1 as 10. It consists of two independent noise canceling systems, such as 11 (only one is shown in the figure), each of which has a residual microphone 12 located at the ear to sense any remaining noise, a system controller 13 to synthesize the anti-noise signal and a headset driver to deliver the anti-noise to the vicinity of the ear. The system components: an LMS (Least Means Square) adapter 14, a adaptive filter 15 and a equalizer 16.

The headset 17 has an open back. This is the most desirable configuration since it is the lightest in weight and, given the selective nature of our noise canceling algorithms, allows the wearer to hear warning signals and external communications thereby increasing user safety.

It has anti-noise speakers 18 to generate sound to cancel unwanted environment noise.

The algorithm used is the digital virtual earth algorithm which develops a reference signal by subtracting a equalized version of its own anti-noise signal from the residual signal. It has been shown to be highly effective in headsets for simple noise environments (a small number of harmonics) even when the noise signal is rapidly varying (as in a siren noise canceling headset). The least Means Square (LMS) adaption shown is a Filtered-X version which has inherent compensation for the effects of the feedback delays around the loop.

The headset driver (or speaker) is capable of producing the anti-noise at the same level as the noise to be cancelled. It has little or no distortion and has a minimum of input-to-output delay as any delay in the feedback loop slows down the system adaptation rate.

The residual microphone 12 is typically a small electret (capacitive) microphone that is mounted on the driver frame 19 near the ear. It faithfully reproduces the sound that remains at the ear after cancellation so that the controller can make further adjustments to the anti-noise signal.

An example of a application of this headset is in emergency vehicles which use high level sirens to alert others of their approach. The modern electronic siren drives a compression type horn speaker with a square wave which rapidly varies in frequency. In the US the square wave varies from 700 to 1500 Hz and has several harmonics. The driver and passengers in the emergency vehicle are also exposed to this noise although the higher harmonics are greatly reduced in amplitude by the vehicle cab.

The noise creates several problems which include hearing damage, safety concerns and intelligible communications.

The risk of hearing damage to the occupants is real since the noise can easily exceed 85 dBA in the cab. The instant invention reduces that by 15 to 20 db for the user.

It is difficult for the driver to hear other sounds (especially other sirens) when the siren is active. This can lead to an increased risk of accidents. The instant invention allows for selective cancellation allowing other noises to be heard more easily with the headset on than with it off.

The siren sound lowers the intelligibility of all communications within the cab and through any communications system. With the instant invention the user is now in a quieter environment. Communications from others in the vehicle or from a radio speaker are easier to hear. If still better intelligibility is desired the communications can be played through the same headset drivers (speakers) that are used to generate the anti-noise.

Insofar as the intelligibility of the outgoing communications/speech is concerned other solutions have been used. These include the close talking microphone which has been used traditionally. The microphone is built to be highly selective and acoustically cancels all sounds outside of its near field. The user either holds it close to his mouth while speaking or it is mounted in front of the mouth by a the headset. the other solution to the problem has been the use of an "in-wire" technique. Here a third channel of noise cancellation is applied to the outgoing signal.

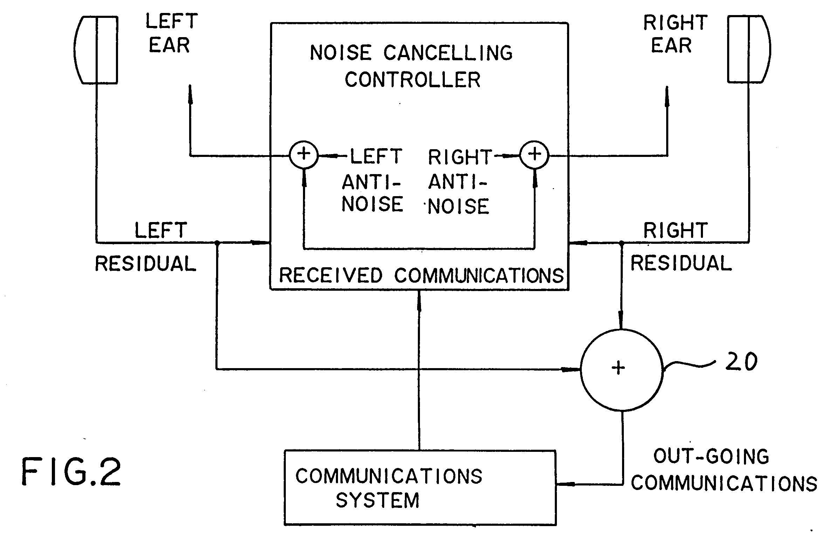

In the instant invention, the solution is cheaper, has no third channel, and is easier to use than a boom mounted microphone. Fig. 2 shows a diagrammatic flow chart of the headset system 10.

The system adds the two residual signals together at 20 and tends to improve the signal to noise in the out-going communications since the two speech signals tend to be in phase and the noise signals have random phase in the two residual signals.

The residual microphones are in a quiet environment due to the operation of the cancellation algorithm. They also respond to the wearer's speech since the open-back headset 17 does not attenuate speech. Therefore the residual signals can be used at the input for outgoing communications.

Thus the noise canceling headset and communications systems are fully integrated.

While only one embodiment of the instant invention has been shown it will be obvious to those of ordinary skill in the art that changes and modifications can be made without departing from the scope of the appended claims.

## Claims

1. A hands-free active noise canceling headset (10) comprising clamping means (17) adapted to secure the headset to the users head, said headset including a speaker means (18) and a microphone means (12) operatively connected to a controller means (13) adpated to produce an anti-noise signal to said speaker means,
characterized in that
the headsets (19) are open backed, the microphone means (12) are residual microphone means directly exposed to the sound originating from the speaker means (18), and in that the signals from said residual microphone means (12) are used for outward bound communications.

2. A headset (10) as in claim 1, characterized in that there are two open backed headsets (19) on that clamping means (17) positioned opposite one another so as to be on opposite sides of a users head over the ears.

3. A headset (10) as in claim 1 or 2, characterized in that said controller means (13) includes an LMS adapter (14) having inherent compensation for any effects or feedback delays around the control circuit, an adaptive filter means (15) and an equalizer means (16) to handle the anti-noise signals and communication signals from said residual microphone means.

4. A headset (10) as in claim 3, characterized in that said controller means (13) is adapted to develop a reference signal by subtracting an equalized anti-noise signal of its own from the residual microphone signal.

5. A headset (10) as in any of the foregoing claims, characterized in that said residual microphone means (12) is a small electret microphone.

6. A headset (10) as in any of the foregoing claims, characterized in that said open backed headset means includes two residual microphones (12) adapted to produce in phase signals and in that the control means adds the signals generated by said two microphones (12) which improves the signal to noise ratio as the signals are in place.

7. A headset (10) as in claim 1, characterized in that the headset means (10) and the control means (13) are connected by wire means.

## Patentansprüche

1. Aktiv Geräusche unterdruckendes, die Hände freilassendes Kopfhörersystem (10), das Haltemittel (17) umfaßt, die geeignet sind, Kopfhörer an dem Kopf eines Benutzers zu halten, der Lautsprechermittel (18) und Mikrophonmittel (12) enthält, welche mit einer Steuerung (13) verbunden sind, die dazu dient, ein Anti-Geräusch-Signal für die Lautsprechermittel zu erzeugen,
**dadurch gekennzeichnet, daß**
die Kopfhörer (19) offene Rückseiten aufweisen, die Mikrophonmittel (12) Restmikrophone darstellen, welche direkt den von den Lautsprechermitteln (18) herrührenden Schallsignalen ausgesetzt sind, und daß die Signale der Restmikrophone (12) zur nach außen bestimmten Kommunikation verwendet werden.

2. Kopfhörersystem (10) nach Anspruch 1, dadurch gekennzeichnet, daß an dem Haltemittel (17) zwei offene Rückseiten aufweisende Kopfhörer (19) angeordnet sind, welche einander gegenüberliegend angeordnet sind, um auf gegenüberliegenden Seiten des Kopfes eines Benutzers über den Ohren angeordnet zu werden.

3. Kopfhörersystem (10) nach Anspruch 1 oder 2, dadurch gekennzeichnet, daß die Steuerung (13) einen LMS-Adapter (14) mit inhärenter Kompensation für jegliche Rückkopplungs- oder Verzögerungseffekte im Controller-Schaltkreis, ein adaptives Filter (15) und einen Equalizer (16) enthält, um die Anti-Geräusch-Signale und Kommunikationssignale, welche von den Restmikrophonen herrühren, zu verarbeiten.

4. Kopfhörersystem (10) nach Anspruch 3, dadurch gekennzeichnet, daß die Steuerung (13) dazu dient, ein Referenzsignal zu erzeugen, indem ein ausgeglichenes Anti-Geräusch-Signal von dem Restmikrophon-Signal subtrahiert wird.

5. Kopfhörersystem (10) nach einem der vorherigen Ansprüche, dadurch gekennzeichnet, daß das Restmikrophon (12) ein kleines Elektret-Mikrophon darstellt.

6. Kopfhörersystem (10) nach einem der vorherigen Ansprüche, dadurch gekennzeichnet, daß das offene Rückseiten aufweisende Kopfhörersystem zwei Restmikrophone (12) enthält, welche dazu dienen, einander gleichphasige Signale zu erzeugen, und daß die Steuerung die von den beiden Mikrophonen (12) erzeugten Signale addiert, was das Signal-Rausch-Verhältnis verbessert.

7. Kopfhörersystem (10) nach Anspruch 1, dadurch gekennzeichnet, daß das Kopfhörersystem (10) und die Steuerung (13) durch Kabel miteinander verbunden sind.

## Revendications

1. Casque mains libres à suppression de bruit active (10) comprenant un moyen de fixation (17) conçu pour maintenir le casque sur la tête de l'utilisateur, ledit casque comportant un moyen de haut-parleurs (18) et des moyens de microphones (12) fonctionnellement reliés à un moyen de contrôleur (13) conçu pour produire un signal antibruit vers ledit moyen de haut-parleurs, caractérisé en ce que les écouteurs (19) sont à ouverture arrière, les moyens de microphones (12) sont des moyens de microphones résiduels directement exposés aux sons provenant des moyens de haut-parleurs (18), et en ce que les signaux provenant desdits moyens de microphones résiduels (12) sont utilisés pour des communications vers l'extérieur.

2. Casque (10) selon la revendication 1, caractérisé en ce qu'il existe deux écouteurs à ouverture arrière (19) sur le moyen de fixation (17), positionnés face à face afin d'être de chaque côté de la tête de l'utilisateur sur les oreilles.

3. Casque (10) selon l'une des revendications 1 ou 2, caractérisé en ce que ledit moyen de contrôleur (13) comporte un adaptateur (14) utilisant la méthode des moindres carrés (LMS) et possédant une compensation interne vis-à-vis de tout effet, ou retard de rétroaction, se produisant dans le circuit de commande, un moyen de filtre adaptatif (15) et un moyen d'égaliseur (16) destinés à traiter les signaux antibruit et les signaux de communications provenant desdits moyens de microphones résiduels.

4. Casque (10) selon la revendication 3, caractérisé en ce que ledit moyen de contrôleur (13) est conçu pour engendrer un signal de référence en soustrayant du signal de microphone résiduel un signal antibruit égalisé qui lui est propre.

5. Casque (10) selon l'une quelconque des revendications précédentes, caractérisé en ce que ledit moyen de microphone résiduel (12) est un petit microphone à électret.

6. Casque (10) selon l'une quelconque des revendications précédentes, caractérisé en ce que lesdits moyens d'écouteurs à ouverture arrière comprennent deux microphones résiduels (12) conçus pour produire des signaux en phase et en ce que le moyen de commande ajoute les signaux engendrés par lesdits deux microphones (12) ce qui améliore le rapport signal sur bruit lorsque les signaux sont présents.

7. Casque (10) selon la revendication 1, caractérisé en ce que le moyen de casque (10) et le moyen de commande (13) sont reliés à l'aide de moyens de fils.
